# EUROPEAN PATENT APPLICATION

(11) **EP 4 644 556 A1**
(43) Date of publication of application: **05.11.2025**
(21) Application number: 23912218.7
(22) Date of filing: 27.12.2023
(51) Int. Cl.: C12N 15/864, C12N 7/01, C12N 15/35

(54) **METHOD FOR PRODUCING ADENO-ASSOCIATED VIRUS, AND VECTOR SET**

(30) Priority: 28.12.2022 JP 2022211032
(71) Applicant: FUJIFILM Corporation, Tokyo 106-8620 (JP)
(72) Inventor: MURAGUCHI, Taichi, Ashigarakami-gun, Kanagawa 258-8577 (JP); MORI, Yusuke, Ashigarakami-gun, Kanagawa 258-8577 (JP); NISHIKAWA, Hiroki, Ashigarakami-gun, Kanagawa 258-8577 (JP)
(74) Representative: Hoffmann Eitle
(86) International application number: PCT/JP2023/046847
(87) International publication number: WO 2024/143440

(57) **Abstract**

An object of the present invention is to provide a method for manufacturing an adeno-associated virus, which is capable of improving a ratio of an amount of a capsid containing a full-length gene to a total amount of the capsids, and a set of vectors for use in the method for manufacturing an adeno-associated virus. According to the present invention, there is provided a method for manufacturing an adeno-associated virus, the method including a gene transfer step of transferring a first vector containing a Rep gene and a Cap gene and a second vector containing a Rep gene and a Cap gene into a cell and a cell culture step of culturing the cell into which gene transfer has been performed, in which the first vector is different from the second vector, the Rep genes contained in the first vector and the second vector are full-length and of the same serum type, and the Cap genes contained in the first vector and the second vector are full-length and of the same serum type.

## Description

### Technical Field

The present invention relates to a method for manufacturing an adeno-associated virus using two types of vectors containing a Rep gene and a Cap gene. The present invention further relates to a set of vectors for manufacturing an adeno-associated virus.

### Background Art

A gene therapy method has been developed in which a recombinant adeno-associated virus (AAV) vector into which a therapeutic gene has been incorporated is administered to a patient for the purpose of treatment of an intractable disease such as a genetic disease, cancer, or the like. As a method of producing an AAV vector, a method of transferring DNA encoding a protein required for AAV production into a cell and allowing the AAV to be produced in the cell has been used.

Patent Document 1 describes a method of packaging recombinant adeno-associated virus (AAV) particles, the method including bringing cells expressing a Rep gene of a first serum type into contact with a recombinant nucleic acid containing a pair of inverted terminal repeats (ITRs) of a second serum type. Patent Document 2 describes a nucleic acid molecule containing (i) a promoter, (ii) a Cap gene, and (iii) a Rep gene in the order of 5' → 3', in which both the Cap gene and the Rep gene are functionally associated with the promoter, and the Rep gene is also functionally associated with IRES. Patent Document 3 describes that the REP is divided into two parts, and mutations are introduced into the promoter region to prevent leaky expression from P19 of Small REP-IRES-Large REP and Large REP. Patent Document 4 describes that a nucleic acid construct encoding REP78 and REP52 is used in the production of adeno-associated virus (AAV) particles. Patent Document 5 describes an insect cell containing a first nucleotide sequence and a second nucleotide sequence, in which the first nucleotide sequence encodes an amino acid sequence of a parvovirus REP52 or REP40 protein, and the second nucleotide sequence encodes an amino acid sequence of a parvovirus REP78 or REP68 protein.

### Prior Art Documents

### Patent Documents

Patent Document 1: JP2021-514659A
Patent Document 2: JP2021-510496A
Patent Document 3: WO2019/057691A
Patent Document 4: JP2022-512621A
Patent Document 5: JP2010-534472A

### SUMMARY OF THE INVENTION

### Object to be solved by the invention

In the method of producing AAV particles by transferring a plasmid containing a Rep gene and a Cap gene, a plasmid containing a virus helper gene, and a plasmid containing a gene for treatment or prevention into HEK293 cells, there is an issue that the expression ratio of REP and CAP at the protein level is not optimized, and a large amount of particles (also referred to as Empty particles) not containing the gene for treatment or prevention are produced. The difference between the Empty particles and the particles (also referred to as Full particles) containing a gene for treatment or prevention is only whether or not the gene for treatment or prevention is encompassed in the particles, and it is difficult to separate and purify the Full particles. In addition, it has been suggested that the Empty particles enhance the immune response at the time of administration and cause side effects, and it is required to reduce the Empty particles. Furthermore, even in the purification step during the production, in a case where the amount of Empty particles is large, the production cost and the purification cost increase. Therefore, it is required to reduce the Empty particles.

An object of the present invention is to provide a method for manufacturing an adeno-associated virus, in which a ratio of an amount of a capsid containing a full-length gene (amount of Full particles) to a total amount of capsids (total amount of Empty particles and Full particles) can be improved. Another object of the present invention is to provide a set of vectors for use in the method for manufacturing an adeno-associated virus.

### Means for solving the object

As a result of intensive studies to achieve the above objects, the inventors of the present invention have found that the ratio of the amount of the capsid containing a full-length gene to the total amount of the capsid can be improved by transferring a first vector containing a Rep gene and a Cap gene and a second vector containing a Rep gene and a Cap gene (here, the first vector is different from the second vector) into cells. The present invention has been completed based on the above findings.

According to an aspect of the present invention, the following inventions are provided.
<1> A method for manufacturing an adeno-associated virus, the method comprising: a gene transfer step of transferring a first vector containing a Rep gene and a Cap gene and a second vector containing a Rep gene and a Cap gene into a cell; and a cell culture step of culturing the cell into which gene transfer has been performed, in which the first vector is different from the second vector, the Rep genes contained in the first vector and the second vector are full-length and of the same serum type, and the Cap genes contained in the first vector and the second vector are full-length and of the same serum type.
<2> The method according to <1>, in which a promoter contained in the first vector is different from a promoter contained in the second vector.
<3> The method according to <1> or <2>, in which a promoter of the first vector is a wild-type promoter of an adeno-associated virus.
<4> The method according to <3>, in which the promoter of the first vector is a P5 promoter, a P19 promoter, or a P40 promoter.
<5> The method according to <1> or <2>, in which a promoter of the second vector is a non-adeno-associated virus-type promoter.
<6> The method according to <1> or <2>, in which a promoter of the second vector is a CMV promoter, a PGK promoter, or an EF1 promoter.
<7> The method according to <1> or <2>, in which the second vector further contains an IRES sequence and/or a 2A self-cleaving peptide sequence between the Cap gene and the Rep gene.
<8> The method according to <1> or <2>, in which the second vector is a vector containing a CMV promoter, a Cap gene, an EMCV sequence, and a Rep gene in this order.
<9> The method according to <1> or <2>, in which the first vector is a vector in which a production amount of a short form of a REP protein is larger than a production amount of a long form of the REP protein, and the second vector is a vector in which a production amount of a long form of a REP protein is larger than a production amount of a short form of the REP protein.
<10> The method according to <1> or <2>, in which a use amount of the second vector is 5% or more with respect to a total use amount of the first vector and the second vector.
<11> The method according to <1> or <2>, in which the first vector and the second vector are plasmid vectors.
<12> The method according to <1> or <2>, in which in the gene transfer step, a third vector containing a virus helper gene and a fourth vector containing a gene for treatment or prevention are transferred into the cells.
<13> The method according to <1> or <2>, in which the cell is a HEK293 cell.
<14> A set of vectors for manufacturing an adeno-associated virus, the set at least comprising: a first vector containing a Rep gene and a Cap gene; and a second vector containing a Rep gene and a Cap gene, in which the first vector is different from the second vector, the Rep genes contained in the first vector and the second vector are full-length and of the same serum type, and the Cap genes contained in the first vector and the second vector are full-length and of the same serum type.

### Effect of the invention

According to the present invention, in the manufacturing of an adeno-associated virus, it is possible to improve a ratio of an amount of the capsid containing the full-length gene to the total amount of the capsids.

### BRIEF DESCRIPTION OF THE DRAWINGS

Fig. 1 shows measurement results of genome titer for Experimental Example 1.
Fig. 2 shows measurement results of a Full ratio for Experimental Example 1.
Fig. 3 shows measurement results of genome titer for Experimental Example 2.
Fig. 4 shows measurement results of a Full ratio in Experimental Example 2.
Fig. 5 shows measurement results of genome titer in Experimental Example 3.
Fig. 6 shows measurement results of a Full ratio in Experimental Example 3.
Fig. 7 shows measurement results of genome titer for Experimental Example 4.
Fig. 8 shows measurement results of a Full ratio in Experimental Example 4.
Fig. 9 shows the results of detecting REP expression by Western blotting.

### EMBODIMENTS FOR CARRYING OUT THE INVENTION

Hereinafter, an example of the embodiment of the present disclosure will be described. However, the present disclosure is not limited to the following embodiments, and can be implemented with appropriate changes within the scope of the object of the present disclosure. In the present specification, the numerical range indicated by using "to" means a range including numerical values described before and after "to" as a minimum value and a maximum value, respectively.

The present invention relates to a method for manufacturing an adeno-associated virus, the method including a gene transfer step of transferring a first vector containing a Rep gene and a Cap gene and a second vector containing a Rep gene and a Cap gene into a cell and a cell culture step of culturing the cell into which gene transfer has been performed, in which the first vector is different from the second vector, the Rep genes contained in the first vector and the second vector are full-length and of the same serum type, and the Cap genes contained in the first vector and the second vector are full-length and of the same serum type.

The Rep gene included in the first vector and the second vector and the Cap gene included in the first vector and the second vector are each full-length. The full length means a sequence from the initiation codon (a codon encoding Met) to the termination codon of the Rep gene.

The Rep gene included in the first vector and the second vector and the Cap gene included in the first vector and the second vector are each same serum type. The same serum type means that the serum types of AAV derived from the Rep gene and the Cap gene are the same.

In the present invention, the Rep gene and the Cap gene, which are genes required for the production of adeno-associated virus (AAV), are transferred into cells using two types of vectors (a first vector and a second vector). By using two types of vectors, it has been possible to separately control gene expression by a promoter, thereby adjusting the protein expression level and producing Full particles at a high ratio. Furthermore, in the present invention, the improvement of the virus titer can be achieved by adjusting the ratio of the first vector to the second vector.

In the related art, an example in which Large REP, which is a starting point for DNA replication, and Small REP, which is responsible for packaging, are each divided by focusing on splicing variants of REP proteins has been reported. However, in the related art, the expression level of the full-length REP is not adjusted, and the expression level of the CAP is not taken into consideration.

### (1) Pre-culture of cells

The cells are not particularly limited, but are preferably animal cells, more preferably mammalian cells or insect cells, and still more preferably mammalian cells. Examples of the mammalian cell include a human cell, a mouse cell, a rat cell, a monkey cell, and a hamster cell, but the cells are not particularly limited. The human cell can be preferably used. Examples of the cell include mouse myeloma (NSO) cell lines, Chinese hamster ovary (CHO) cell lines, HT1080, H9, HepG2, MCF7, MDBK Jurkat, NIH3T3, PC12, a baby hamster kidney (BHK) cell, VERO, SP2/0, YB2/0, Y0, C127, an L cell, COS (for example, COS1 and COS7), QC1-3, a human embryo-derived kidney (HEK293) cell, VERO, PER. C6, HeLa, EB1, EB2, EB3, and oncolytic or hybridoma cell lines. The cell is preferably a HEK293 cell or a CHO cell, and more preferably a HEK293 cell. The HEK293 cells are cells into which an E1A gene and an E1B gene derived from an adenovirus are incorporated. The cells may be either adherent cells or suspension cells, but suspension cells are preferable to perform high-concentration large-scale culture in a reactor.

As a cell line having high cell proliferation properties and high AAV production ability per cell, for example, a suspension HEK293 cell line Viral Production Cells 2.0 (also referred to as VPCs 2.0) manufactured by Thermo Fisher Scientific, Inc. can be used.

In addition, at least one or more selected from a gene for treatment or prevention, E2, E4, or VA-RNA among gene code groups required for AAV production described later may be incorporated in advance into the genome of the cell (packaging cell).

As the culture medium, a culture medium optimized for culturing HEK293 cells based on PBS is available commercially by each company. A Balan-CD HEK293 culture medium manufactured by FISI can be used. Additional component may be appropriately supplemented to the culture medium as necessary. Examples of the additional component include an amino acid, a salt, sugars, a vitamin, a hormone, a proliferation factor, a lipid, a trace element, and the like, but the additional component is not particularly limited. The pH of the culture medium is 6 to 8, preferably 6.8 to 7.6, and more preferably 7 to 7.4.

The method of pre-culture of cells is not particularly limited, and may be any of batch culture, fed-batch culture, perfusion culture, shaking culture, stirring culture, standing culture, or adhesion culture. From the viewpoints of large-scale treatment and high concentration, perfusion culture is preferable. In the perfusion culture, the removal of waste components by a filter and the supply of a fresh culture medium make it possible to perform proliferation at a high concentration by maintaining the quality (component concentration) of the suspension under conditions suitable for cell proliferation.

The temperature of the pre-culture of the cells is preferably 30°C to 40°C, more preferably 32°C to 37°C, and particularly preferably 36°C to 37°C.

The cell density is preferably 1 × 10⁶ to 20 × 10⁶ cells/mL in the batch culture, preferably 1 × 10⁶ to 50 × 10⁶ cells/mL in the fed-batch culture, and preferably 1 × 10⁶ to 200 × 10⁶ cells/mL in the perfusion culture (dynamic/continuous).

The culture time of the pre-culture is preferably 12 hours or more.

In a case of perfusion culture, either a dynamic method or a continuous method may be used. In the dynamic method, cells are seeded at a low concentration, proliferative culture is performed, the total amount of the cell solution is recovered after reaching a high concentration, and gene transfer is performed. In the continuous method, cells are seeded at a low concentration, proliferated culture is performed, a certain amount of cells are extracted (cell bleeding) after reaching a certain concentration, and gene transfer is performed. Fresh culture medium in the same amount as the extraction amount is supplemented on the reactor side to maintain the constant cell concentration in a reactor. The cell bleeding and the supply of the culture medium may be continuous or intermittent (for example, once a day at 10% to 50% or the like). The concentration is recovered by cell proliferation until the next cell bleeding. The above operation can be repeated, for example, for 1 day to 3 months.

### (2) Adjustment of cell concentration and exchange of culture medium

The cell concentration at the time of gene transfer is preferably 1 × 10⁶ cells/mL to 100 × 10⁶ cells/mL, more preferably 1 × 10⁶ cells/mL to 50 × 10⁶ cells/mL, and still more preferably 2 × 10⁶ cells/mL to 10 × 10⁶ cells/mL.

The cell concentration may be increased as compared with the cell concentration at the time of the pre-culture, by cell concentration using centrifugation, continuous centrifugation, sonic agglomeration, acoustic electrophoresis, filtration (TFF), or the like.

### (3) Gene to be transferred into cell

In the present invention, a first vector containing a Rep gene and a Cap gene and a second vector containing a Rep gene and a Cap gene are transferred into cells.

Adeno-associated virus (AAV) refers to a small, incompletely replicative, non-enveloped virus containing single-stranded DNA having about 4,700 bases, from the family of Parvoviridae and Dependoparvovirus. It is known that there are more than 100 serum types in AAV, and that the host range and the characteristics of the virus differ depending on the difference in the serum types. Serum type 2 (AAV2) is one of the serum types that have been widely studied for a long time, and it is known that the host range is very wide. The serum type 1 (AAV1), the serum type 5 (AAV5), and the serum type 6 (AAV6) are serum types having higher tissue tropisms. It is said that AAV1 has high gene transfer efficiency into muscle, liver, airway, central nervous system, and the like, AAV5 has high gene transfer efficiency into central nervous system, liver, retina, and the like, and AAV6 has high gene transfer efficiency into heart, muscle, liver, and the like. In the present invention, it is preferable to use a Rep gene and a Cap gene derived from AAV2, AAV5, AAV6, or AAV8.

The adeno-associated virus gene refers to a gene composed of one or a plurality of nucleic acid sequences derived from the serum types of one or a plurality of adeno-associated viruses. The adeno-associated virus gene is specifically a Rep gene and a Cap gene. The Rep gene is a gene involved in the replication and packaging of AAV. The Cap gene is a gene encoding an AAV constituent protein.

AAV is a non-enveloped virus that proliferates in the presence of helper viruses such as adenovirus and herpesvirus. In the preparation of AAV to be used for gene therapy or nucleic acid transfer, classically, AAV replication has been performed by co-infecting host cells with an adenovirus. In addition, a gene responsible for the helper action of adenovirus has been clarified, and a plasmid incorporating this gene has also been used. For example, a plasmid containing a Rep gene and a Cap gene, an adenovirus helper plasmid, and a plasmid containing a gene for treatment or prevention are simultaneously transfected into cells, and can thus be packaged as recombinant AAV (rAAV).

The Rep gene and the Cap gene encode proteins involved in the replication and packaging of the virion. In the wild type, the Rep gene is expressed from the p5 promoter and the p19 promoter. The Cap region expresses VP1, VP2, and VP3. Examples of the promoter that is naturally carried by the Cap gene can include a p40 promoter.

The adeno-associated virus gene (a Rep gene and a Cap gene) may be a wild-type gene, but a gene in which modifications such as substitution, deletion, insertion, or addition of a base have been made to the wild-type gene may be used as long as it exhibits an inherent function.

In a case where modifications such as substitution, deletion, insertion, or addition of a base are made to the wild-type adeno-associated virus gene (a Rep gene and a Cap gene), the number of the modified bases is preferably 1 to 20, more preferably 1 to 10, and still more preferably 1 to 3. The modified base sequence of the adeno-associated virus gene exhibits preferably 85% or more sequence identity, more preferably 90% or more sequence identity, still more preferably 95% or more sequence identity, and even still more preferably 98% or more sequence identity with the base sequence of the wild-type adeno-associated virus gene.

In a case of transferring the Rep gene and the Cap gene into cells, a vector containing the Rep gene and the Cap gene is transferred into cells. The arrangement of the Rep gene and the Cap gene in a vector is not particularly limited, the Rep gene may be located upstream of the Cap gene or downstream of the Cap gene.

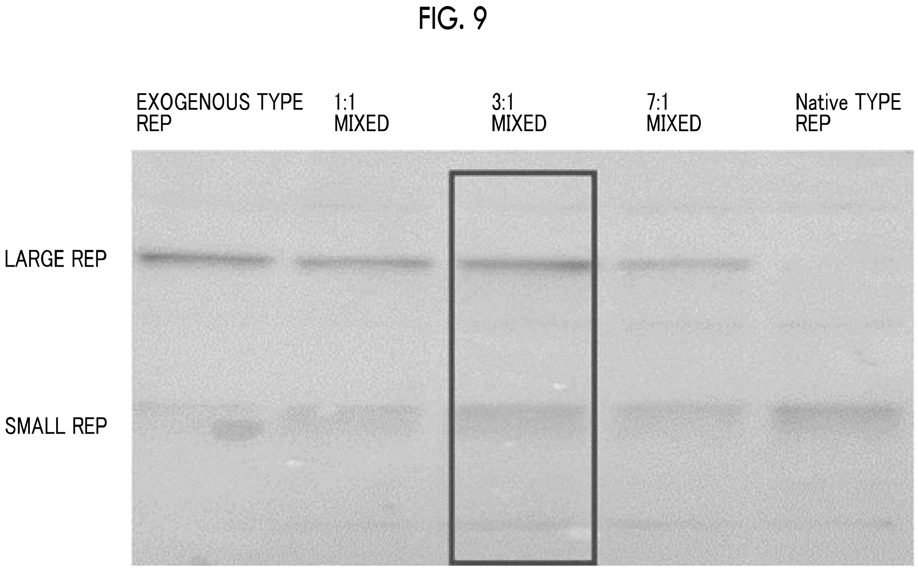

The Rep gene means a region of the AAV genome encoding a virus replication protein that is known to those skilled in the art and that is collectively required for replication of a virus genome, or its functional homolog, for example, a human herpesvirus 6 (HHV-6) Rep gene, or the like (mediating AAV-2 DNA replication is known). Therefore, the coding region of the Rep gene includes at least genes encoding REP78 and REP68 (also referred to as a long form of Rep protein; represented by large Rep in Fig. 9) of AAV, and REP52 and REP40 (also referred to as a short form of Rep protein; represented by small Rep in Fig. 9) of AAV, or functional homologs thereof. The coding region of the Rep gene used in the present invention may be derived from any AAV serum type, but is preferably derived from AAV2. Examples of those derived from AAV2 include REP78 and REP68, and REP52 and REP40, and ITR.

The Cap gene means a region in the AAV genome encoding a capsid protein of a virus known to those skilled in the art. Examples of these capsid proteins are AAV capsid proteins VP1, VP2 and VP3. The Cap gene used in the present invention may be derived from any AAV serum type, but is preferably derived from AAV2, AAV5, AAV6, or AAV8.

As the first vector containing a Rep gene and a Cap gene and the second vector containing a Rep gene and a Cap gene, for example, a plasmid vector, a vector containing a nucleic acid sequence derived from a virus, a vector containing an artificially designed nucleic acid, or the like can be used, and a plasmid vector is preferable.

In the present invention, a promoter contained in the first vector is preferably different from a promoter contained in the second vector.

The promoter of the first vector is preferably a wild-type promoter of an adeno-associated virus, and more preferably a P5 promoter, a P19 promoter, or a P40 promoter.

The promoter of the second vector is preferably a non-adeno-associated virus-type promoter, and more preferably a CMV promoter, a PGK promoter, or an EF1 promoter.

The second vector preferably further contains an IRES sequence and/or a 2A self-cleaving peptide sequence between the Cap gene and the Rep gene.

The second vector is preferably a vector containing a CMV promoter, a Cap gene, an EMCV sequence (preferably, EMCV IRES), and a Rep gene in this order. Here, EMCV IRES means picornavirus (encephalomyocarditis virus) IRES.

It is preferable that the first vector is a vector in which a production amount of a short form of a REP protein is larger than a production amount of a long form of the REP protein and the second vector is a vector in which a production amount of a long form of a REP protein is larger than a production amount of a short form of the REP protein. By optimizing the production amount of the long form of the REP protein and the short form of the REP protein, the ratio of the Full particles can be improved.

In the first vector, the production amount of the short form of the REP protein is preferably 51% or more, more preferably 60% or more, still more preferably 80% or more, and even still more preferably 90% or more, with respect to the total production amount of the long form of the REP protein and the short form of the REP protein.

In the second vector, the production amount of the long form of the REP protein is preferably 51% or more, more preferably 60% or more, still more preferably 70% or more, and even still more preferably 80% or more, with respect to the total production amount of the long form of the REP protein and the short form of the REP protein.

Regarding the production amount of the long form of the REP protein and the short form of the REP protein, only the first vector or only the second vector is prepared, and the production amount of the REP protein can be compared by transferring the vector together with the vector containing the helper gene into the cells and performing Western blotting in the same manner as in Example 5.

In the present invention, the REP expression may be controlled by using different promoters as promoters that control the expression of REP in the first vector and the second vector and adjusting the ratio of the use amounts of the first vector and the second vector.

As an example of the present invention, a vector containing a Rep gene, a Cap gene, and a P5 promoter in this order can be used as the first vector, and a vector containing a CMV promoter, a Cap gene, an EMCV sequence, and a Rep gene in this order can be used as the second vector. In a case of producing AAV using the first vector and the second vector, as a preferred use amount of the first vector and the second vector, the use amount of the second vector is preferably 5% or more, more preferably 10% or more, still more preferably 40% or more, even still more preferably 50% or more, and yet even still more preferably 60% or more, with respect to the total use amount of the first vector and the second vector. The use amount of the second vector is less than 100%, preferably 95% or less, more preferably 92% or less, and still more preferably 90% or less.

In a case of producing AAV2, the use amount of the second vector is preferably 30% or more, more preferably 40% or more, still more preferably 50% or more, and even still more preferably 55% or more, with respect to the total use amount of the first vector and the second vector. The use amount of the second vector is preferably 95% or less, more preferably 90% or less, and still more preferably 85% or less.

In a case of producing AAV5, the use amount of the second vector is preferably 5% or more, more preferably 7% or more, and still more preferably 10% or more, with respect to the total use amount of the first vector and the second vector. The use amount of the second vector is preferably 50% or less, more preferably 40% or less, and still more preferably 30% or less.

In a case of producing AAV6, the use amount of the second vector is preferably 30% or more, more preferably 40% or more, still more preferably 50% or more, and even still more preferably 55% or more, with respect to the total use amount of the first vector and the second vector. The use amount of the second vector is preferably 95% or less, more preferably 90% or less, and still more preferably 85% or less.

In a case of producing AAV8, the use amount of the second vector is preferably 30% or more, more preferably 50% or more, still more preferably 60% or more, and even still more preferably 70% or more, with respect to the total use amount of the first vector and the second vector. The use amount of the second vector is preferably 95% or less, more preferably 92% or less, and still more preferably 90% or less.

In the present invention, it is preferable that a virus helper gene derived from an adenovirus is transferred into cells. The virus helper gene is a non-adeno-associated virus gene for enabling replication and packaging of an adeno-associated virus. As the virus helper gene, a gene derived from a virus of another species other than the adeno-associated virus is used. Specific examples of the virus helper gene include a virus helper gene derived from an adenovirus or a herpesvirus, and the virus helper gene is preferably derived from an adenovirus.

Examples of the virus helper gene derived from adenovirus can include E1A, E1B, E2A, E4, and VA-RNA. In the host cell having all or a part of the E1 region, the region of the adenovirus genome required for replicating the AAV genome and packaging of the AAV genome into the capsid to form a virus virion is the E2A region, the E4 region, and the VA-RNA region. For the function by the E4 region, the 34 kDa E4 protein encoded by the open reading frame 6 (E4ORF6) of the E4 region is required for replicating the AAV. Preferably, the virus helper gene is an E2 gene, an E4 gene, or a VA-RNA gene. The VA-RNA gene is preferably a VA-RNAI gene.

The adenovirus-derived virus helper gene (such as an E1A, an E1B, an E2A, an E4, and a VA-RNA) may be a wild-type gene, but a gene in which modifications such as substitution, deletion, insertion, or addition of a base have been made to the wild-type gene may be used as long as it exhibits an inherent function.

In a case where modifications such as substitution, deletion, insertion, or addition of a base is made to the wild-type virus helper gene, the number of the modified bases is preferably 1 to 20, more preferably 1 to 10, and still more preferably 1 to 3. The modified base sequence of the virus helper gene exhibits preferably 85% or more sequence identity, more preferably 90% or more sequence identity, still more preferably 95% or more sequence identity, and even still more preferably 98% or more sequence identity with the base sequence of the wild-type virus helper gene.

In a case where the virus helper gene is transferred into cells, a vector containing the virus helper gene (in the present specification, also referred to as a third vector) can be transferred into the cells.

As the vector containing a virus helper gene, for example, a plasmid, a virus-derived sequence, an artificially designed nucleic acid, or the like can be used, and a plasmid is preferable.

The virus helper gene is preferably under the control of a promoter and may be under the control of a promoter capable of regulating expression.

The specific examples of the promoter are not particularly limited, but can include a cytomegalovirus-derived promoter (CMV promoter) (including an enhancer as desired), an SV40 early promoter, a human elongation factor-1α (EF-1α) promoter, a human ubiquitin C promoter, a retrovirus Rous sarcoma virus (RSV) LTR promoter, a dihydrofolate reductase promoter, a β-actin promoter, and a phosphoglycerate kinase (PGK) promoter.

In the present invention, a gene for treatment or prevention may be introduced into cells. It is preferable that the gene for treatment or prevention is transferred in a state of being sandwiched between ITRs.

As the gene for treatment or prevention, a gene that is incomplete or lost in the genome of the target cell, a gene encoding non-natural protein having a desired biological or therapeutic effect (for example, antiviral function), or the like can be used, but the gene is not particularly limited. Specific examples of the gene for treatment or prevention include a gene used for treatment or prevention of inflammatory diseases, autoimmunity, chronic and infectious diseases (including disorders such as acquired immune deficiency syndrome (AIDS), cancer, nervous system diseases, cardiovascular diseases, and hypercholesterolemia), various blood diseases such as anemia and hemophilia, or gene deficiency (for example, cystic fibrosis, Gaucher's disease, adenosine deaminase (ADA) deficiency, emphysema, and the like).

The gene for treatment or prevention may be several antisense oligonucleotides (for example, a short-chain oligonucleotide complementary to a sequence around a translation initiation site (AUG codon) of mRNA) that are useful in antisense therapy against cancer and viral disease.

The gene for therapy or prophylaxis may be linked to a promoter for expressing the gene for treatment or prevention. The promoter for expressing a gene for treatment or prevention is not particularly limited, but examples thereof can include a cytomegalovirus-derived promoter (including an enhancer as desired), an SV40 early promoter, a human elongation factor-1α (EF-1α) promoter, a human ubiquitin C promoter, a retrovirus Rous sarcoma virus LTR promoter, a dihydrofolate reductase promoter, a β-actin promoter, a phosphoglycerate kinase (PGK) promoter, and the like. The gene for treatment or prevention and the promoter for expressing the gene are preferably flanked by the ITR sequences.

As a vector containing a gene for treatment or prevention (also referred to as a fourth vector in the present specification), for example, a plasmid, a virus-derived sequence, an artificially designed nucleic acid, or the like can be used, and a plasmid is preferable.

As described above, in the present invention, preferably, in the gene transfer step, a third vector containing a virus helper gene and a fourth vector containing a gene for treatment or prevention are transferred into the cells. Alternatively, as another form, the first vector containing the Rep gene and the Cap gene or the second vector containing the Rep gene and the Cap gene may contain a gene for treatment or prevention.

The method for manufacturing a virus is a gene transfer method.

### (4) Gene transfer

In the gene transfer step, the first vector and the second vector (and further, the third vector and the fourth vector as desired) are transferred into the cells.

A method of transferring the vector into cells is not particularly limited, and the transfer can be performed by a method such as transfection, transduction, or lipofection.

Transfection refers to transferring a nucleic acid into a cell by crossing a membrane of a eukaryotic cell by a chemical means (for example, a calcium phosphate-mediated precipitation, lipofection, or a method using a polymer such as polyethyleneimine), a mechanical means (for example, electroporation), or a physical means (for example, delivery by bioballistic).

Transduction refers to transfer of a nucleic acid into a cell across a membrane of a eukaryotic cell via a virus-derived vector.

Gene transfer can be performed preferably by transfection and more preferably by using polyethyleneimine. In the polyethyleneimine (PEI) method, a complex of a negatively charged plasmid DNA and a cationic polymer PEI is formed to neutralize the charge, and the PEI-plasmid complex can approach a negatively charged cell, and the complex is transferred into the cell by endocytosis. Then it leads to the endosome escape in the cell, the PEI release, and the gene transcription and translation reaction in the nucleus.

### (5) Culture for virus production

In the present invention, the virus is produced by performing a cell culture step of culturing cells into which a gene has been transferred.

The culture method of the cell culture step is not particularly limited, and may be batch culture, fed-batch culture, or the like, or may be any of shaking culture, stirring culture, standing culture, or adhesion culture.

The culture temperature of the cells is not particularly limited, and the culture of the cells is performed at a temperature at which the cells can survive. The culture temperature is generally 25°C to 45°C, preferably 30°C to 42°C, more preferably 35°C to 40°C, and 37°C as an example. The CO₂ concentration is generally 3 to 10% CO₂, preferably 5 to 10% CO₂, and 8% CO₂ as an example.

The cell density is preferably equal to or less than that in the pre-culture. The cell density is preferably 1 × 10⁶ to 10 × 10⁶ cells/mL in a case of the batch culture, and preferably 1 × 10⁶ to 20 × 10⁶ cells/mL in a case of the fed-batch culture.

It is preferable that the shearing is equal to or less than that of the pre-culture, and it is preferable that the oxygen concentration is equal to or higher than that of the pre-culture. The nutritional components are the same as those in the preculture.

The period of the virus production is preferably 24 hours or more and 30 days or less, more preferably 24 hours or more and 20 days or less, still more preferably 24 hours or more and 10 days or less, even still more preferably 24 hours or more and 7 days or less, and particularly preferably 24 hours or more and 72 hours or less.

As the culture container, a flask or a bioreactor can be used, but it is not particularly limited.

The culture scale is not particularly limited, and the cells can be cultured in a culture medium of any volume, for example, the cells can be cultured in a culture medium of 1 mL to 3,000 L, preferably 1 L to 2,500 L, more preferably 50 L to 2,300 L, and particularly preferably 400 L to 2,200 L.

The culture may be performed while stirring by shaking. The stirring speed in the case of stirring by shaking is generally 50 rpm to 200 rpm, and preferably 80 to 150 rpm. The stirring culture may be rotary stirring culture using a propeller or the like in a reactor. The stirring speed in the case of stirring by rotating is generally 50 rpm to 200 rpm, and preferably 80 to 150 rpm. In addition, stirring may be performed by wave-type shaking stirring or vertical movement of the stirring blade, but the stirring is not particularly limited.

In the present invention, it is preferable that the cells are in a suspension in the gene transfer step and the cell culture step, and the suspension is a culture medium.

### (6) Recovery and purification of virus

The method according to the embodiment of the present invention may preferably include recovering the produced virus. The virus particles produced in the cell are present in the culture solution or in the cell (in the nucleus). The extraction from the inside of the cell (destruction/dissolution of the cell membrane and the nuclear membrane) may be performed by a freeze-thawing method, or may be performed by adding a surfactant (such as Triton X) and stirring. The separation of the virus and the cell debris can be performed by centrifugation or depth filtration.

The virus may be purified by performing TFF (concentration/buffer exchange), affinity chromatography, or AEX (anion exchange chromatography) treatment as necessary. In this manner, it is possible to remove cell debris, cell-derived nucleic acids, and cell-derived proteins. Alternatively, a commercially available virus purification kit (such as an AAV purification kit) may be used. For example, an AAVpro (registered trademark) Purification Kit Maxi/Midi manufactured by Takara Bio Inc. or the like can be used.

### (7) Analysis

The titer of the virus produced by the method according to the embodiment of the present invention can be measured by a normal method known to those skilled in the art. For example, the cell culture solution after the culture is collected, the cells are disrupted by freeze-thaw, and then the supernatant is collected by centrifugation. The gDNA (genomic DNA) and the residual plasmid can be digested by adding MgCl₂ and Benzonase to the collected supernatant to react. It is possible to measure the virus titer (vg/mL) by using the sample containing the target virus obtained as described above as a droplet digital PCR (ddPCR) sample, and performing ddPCR to measure the number of copies of intra-viral genome.

Furthermore, the Full ratio can be calculated from the ratio of the capsid particle titer (Vp/mL) measured using the ELISA kit to the genome titer (vg/mL) calculated by ddPCR measurement. Alternatively, the Full/Empty ratio may be obtained by analyzing the sample solution after virus extraction and purification by HPLC measurement, isoelectric focusing, or the like in the same measurement system.

In the present invention, in the virus produced in the culture step, the ratio (Full ratio) of the amount of the capsid containing the full-length gene to the total amount of the capsid is preferably 5% or more, more preferably 10% or more, still more preferably 20% or more, and particularly preferably 30% or more.

Furthermore, according to the present invention, there is provided a set of vectors for manufacturing an adeno-associated virus, the set at least comprising: a first vector containing a Rep gene and a Cap gene; and a second vector containing a Rep gene and a Cap gene, in which the first vector is different from the second vector, the Rep genes contained in the first vector and the second vector are full-length and of the same serum type, and the Cap genes contained in the first vector and the second vector are full-length and of the same serum type. The details and preferred aspects of each vector are as described in the present specification.

The present invention will be more specifically described using the following examples; however, it is not limited by the examples.

### Examples

The used reagents are shown below.

**[Table 1]**

| Reagent | Vender | Catalog No. |
|---|---|---|
| BalanCD for HEK | FISI | 91165-1L |
| GlutaMAX Supplement | Thermo Fisher | 35050-061 |
| PEIpro | PolyPlus-transfection | 115-010 |
| Triton X-100 Surfact-Amps Detergent Solution | Thermo Fisher | 85111 |
| 1 M MgCl₂ | Fujifilm Wako | 310-90361 |
| Benzonase 99% 10 KU | Merck | 70664-3 |
| GOI plasmid (pGOI) | All synthesized | - |
| RepCap plasmid (pRC) Exogenous type serum type 2, 5, 6, 8 | All synthesized | - |
| RepCap plasmid (pRC) Native type serum type 2, 5, 6, 8 | Takarabio | 6230 |
| Helper plasmid | Takarabio | 6230 |

The base sequence of each plasmid is shown in the following table.
Base sequence of GOI plasmid (pGOI): SEQ ID NO: 1
RepCap plasmid (pRC) Exogenous type serum type 2: SEQ ID NO: 2
RepCap plasmid (pRC) Exogenous type serum type 5: SEQ ID NO: 3
RepCap plasmid (pRC) Exogenous type serum type 6: SEQ ID NO: 4
RepCap plasmid (pRC) Exogenous type serum type 8: SEQ ID NO: 5
RepCap plasmid (pRC) Native type serum type 2: SEQ ID NO: 6
RepCap plasmid (pRC) Native type serum type 5: SEQ ID NO: 7
RepCap plasmid (pRC) Native type serum type 6: SEQ ID NO: 8
RepCap plasmid (pRC) Native type serum type 8: SEQ ID NO: 9
Helper plasmid: SEQ ID NO: 10

It is noted that the pRC Exogenous type contains a CMV promoter, a Cap gene, an EMCV sequence (EMCV IRES), and a Rep gene in this order, and the pRC Native type contains a Rep gene, a Cap gene, and a P5 promoter in this order.

### Experimental Example 1: HEK293 cells: AAV5

### <Preparation of cells>

On the day before transfection, 24 mL of HEK293 cells were seeded at 1.2 × 10⁶ cells/mL in a 125 mL flask. As the culture medium, a culture medium in which GlutaMAX Supplement was added to Balan CD at a final concentration of 2% was used. At the time of transfection at the next day, the cell number reached about 2 × 10⁶ cells/mL, and the viability reached 90% or more.

### <Preparation of transfection complex>

The plasmid was added to a Balan CD culture medium having a volume of 7.5% by volume of the culture volume (1.8 mL in a case of a culture volume of 24 mL) such that the total amount was 1 µg/mL of the culture volume (24 µg in a case of a culture volume of 24 mL).

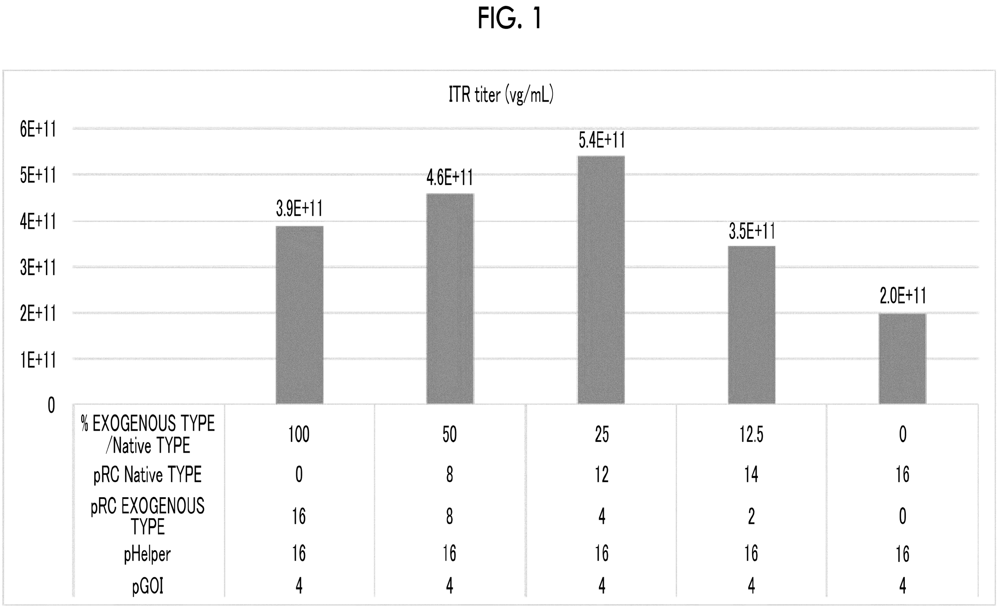

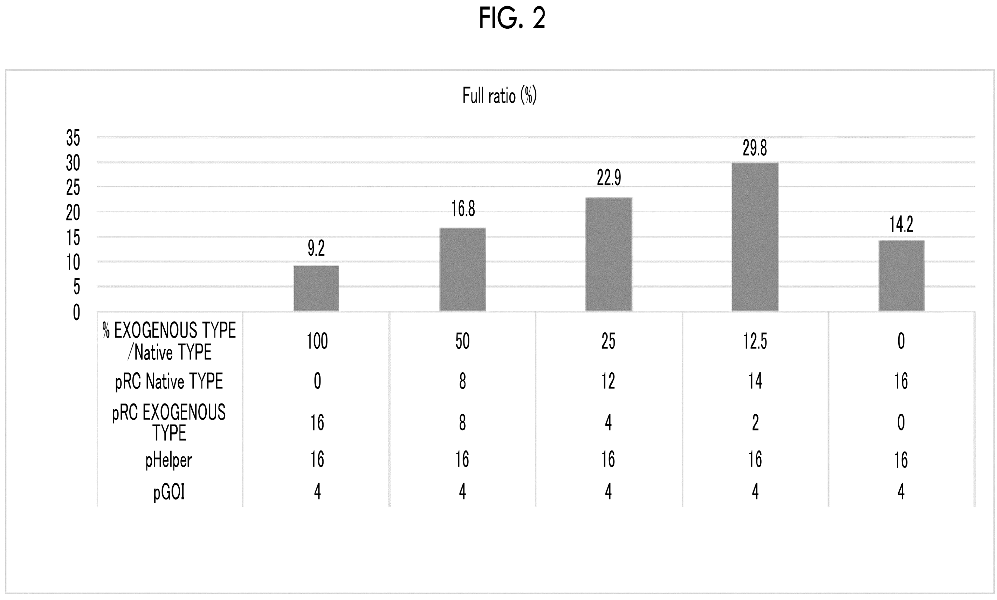

The plasmid was added in the following amounts shown in Figs. 1 and 2.
pGOI (GOI plasmid): 4 µg
pRC Exogenous type (RepCap plasmid): 0 µg, 2 µg, 4 µg, 8 µg, or 16 µg
pRC Native type (Takara RepCap plasmid): 0 µg, 8 µg, 12 µg, 14 µg, or 16 µg
pHelper (Helper plasmid): 16 µg

In another tube, a transfection reagent PEIpro was added to a Balan CD culture medium having a volume of 7.5% of the culture volume (1.8 mL in a case of 24 mL culture) such that the total amount was 3 µL/mL of the culture volume (72 µL in a case of 24 mL culture).

The total amount of the tube containing the transfection reagent was transferred to the tube containing the plasmid, lightly mixed to be uniform, and allowed to stand for 15 minutes.

### <Transfection and culture>

The total amount of the plasmid-transfection reagent complex prepared above was added to the cell culture solution, and the cells were cultured at 37°C, 8% CO₂, and 150 rpm for 72 hours.

### <AAV Recovery for Measuring Genome Titer>

After 72 hours from the transfection, AAV for genome titer measurement was extracted from the cells by the following procedure.

About 2 mL of the culture solution was sampled, and Triton X-100 having a final concentration of 0.1%, 2 mmol/L MgCl₂, and 1.25E-5 U/cell Benzonase were added thereto, and the mixture was reacted at 37°C and 180 rpm. The mixture was centrifuged at 10,185 × g for 10 minutes and the supernatant was recovered. The supernatant was dispensed in 20 µL aliquots and stored at -80°C.

### <Measurement of genome titer>

The sample dispensed and frozen at -80°C was thawed. The sample was heat-treated at 95°C for 15 minutes using a thermal cycler to inactivate the benzonase and dissolve the capsid. Hereinafter, the concentration was quantified by ddPCR targeting the AAV ITR region by the same method as described in Hum Gene Ther Methods. 2019 Aug; 30 (4): 127-136.

### <AAV Recovery for HPLC Measurement>

After 72 hours from the transfection, a sample for using a simple purification kit was prepared for HPLC measurement according to the following procedure.

12 mL of the culture solution was sampled and centrifuged at 1,200 rpm for 5 minutes, and the pellet was recovered. A purification kit (Cat #6666) manufactured by Takara Bio Inc. was used. 2 mL of the extraction solution A attached to the kit was added thereto, and the cells were homogenized by pipetting. After reacting for about 10 minutes, 0.2 mL of the extraction solution B attached to the kit was added. Centrifugation was performed at 1,200 rpm for 5 minutes to recover the supernatant. The reaction of the extraction solution A and the extraction solution B was performed again on the remaining pellets. The supernatant obtained for the second time was mixed with the supernatant obtained for the first time. In the following procedure, a DNase reaction, a precipitator, an Amicon washing, and a concentration step were performed according to the protocol attached to the kit.

### <HPLC measurement>

The full ratio of AAV was measured using a high-performance liquid chromatography method. An anion exchange column was used as a separation column, and a fluorescence detector (excitation: 280 nm, detection: 348 nm) was used for detection. The Full ratio was calculated from the area value of each of the Empty peak and the Full peak of the AAV.

### <Western blotting>

The cells were recovered from 100 µL of the cell culture solution by centrifugation (300 × g). 100 µL of RIPA buffer (FUJIFILM Wako Pure Chemical Corporation, 188-02453) was added to the collected cells, and the cells were re-suspended. Furthermore, 20 µL of Sample Buffer (nacalai tesque, 09499-14) was added thereto, and the mixture was heated at 100°C for 5 minutes to prepare a sample. The protein in the sample was size-separated using a sodium dodecyl sulfate (SDS) gel electrophoresis device (ATTO CORPORATION, 2321670), and then transferred to a membrane using a semi-dry blotting device (ATTO CORPORATION, 2322490). The membrane after the transfer was blocked with Blocking One (nacalai tesque, 03953-95) for 30 minutes, and then reacted with an anti-REP antibody (OriGene Technologies, BM5092) diluted 70-fold with a blocking solution at room temperature for 3 hours. Next, the reaction was performed with the anti-mouse IgG-HRP antibody (Cytiva, NA931-100UL) diluted 10,000 times with a blocking solution at room temperature for 2 hours, and then the REP protein and the β-actin protein were detected by SuperSignal West Femto Maximum Sensitivity Substrate (Thermo Fisher Scientific, Inc., 34095). WSE-6100 LuminoGraphI (ATTO CORPORATION, 2006100) was used for imaging.

For Experimental Example 1, the measurement results of the genome titer are shown in Fig. 1, and the measurement results of the Full ratio are shown in Fig. 2.

### Experimental Example 2: HEK293 cells: AAV2

The cells were prepared in the same manner as in Experimental Example 1.

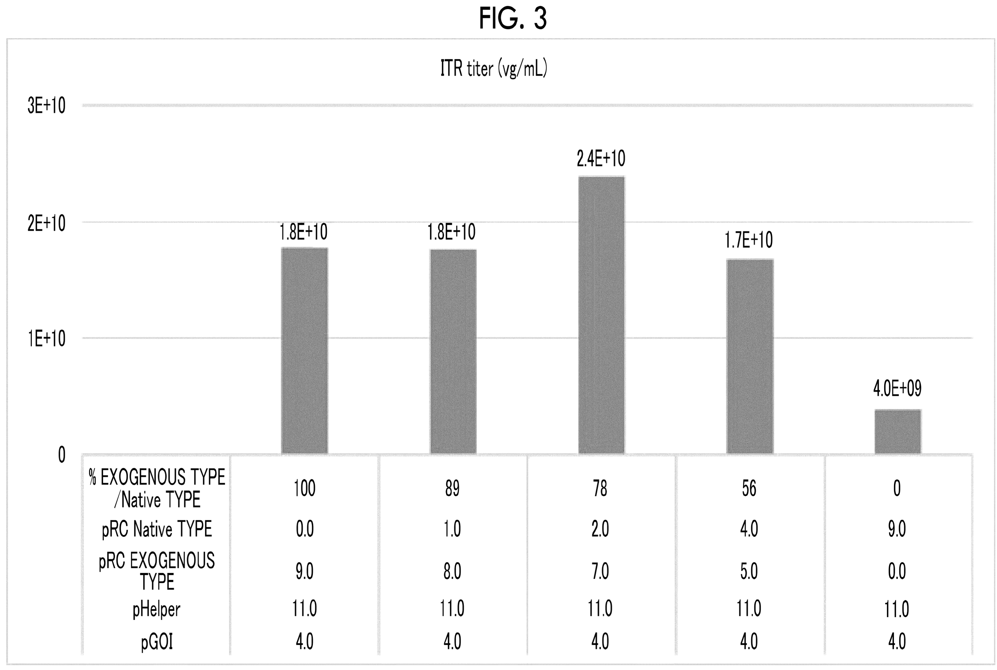

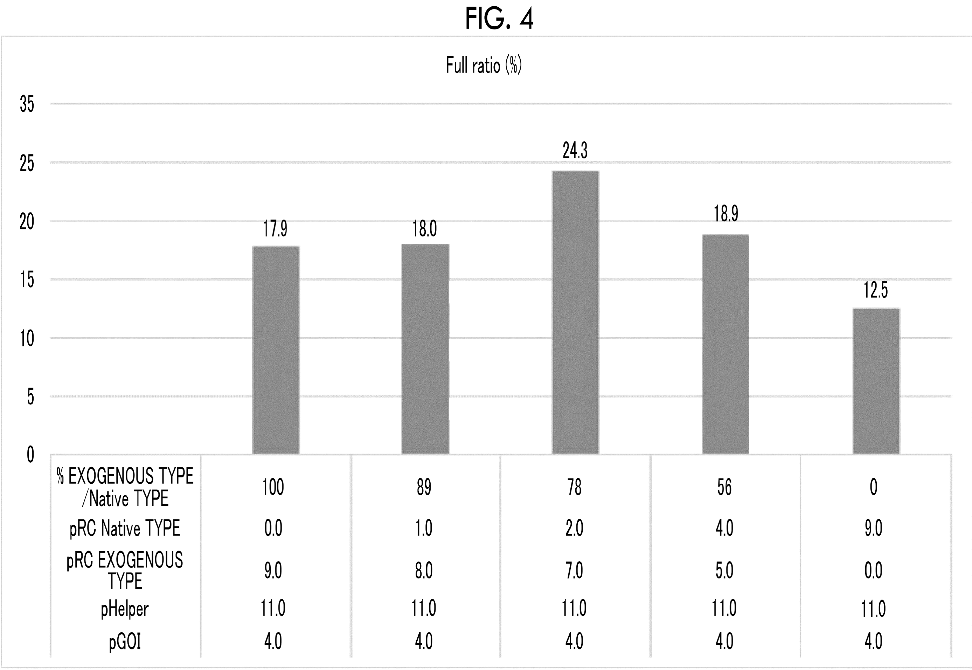

A transfection complex was prepared in the same manner as in Experimental Example 1, except that AAV2 serum type was used as the pRC Native type and the plasmid was added in the following amounts described in Figs. 3 and 4.
pGOI (GOI plasmid): 4 µg
pRC Exogenous type (RepCap plasmid): 0 µg, 5 µg, 7 µg, 8 µg, or 9 µg
pRC Native type (Takara RepCap plasmid): 0 µg, 1 µg, 2 µg, 4 µg, or 9 µg
pHelper (Helper plasmid): 11 µg

Transfection and culture, AAV recovery for measuring genome titer, measurement of genome titer, AAV recovery for HPLC measurement, and HPLC measurement were performed in the same manner as in Experimental Example 1.

For Experimental Example 2, the measurement results of the genome titer are shown in Fig. 3, and the measurement results of the Full ratio are shown in Fig. 4.

### Experimental Example 3: HEK293 cells: AAV6

The cells were prepared in the same manner as in Experimental Example 1.

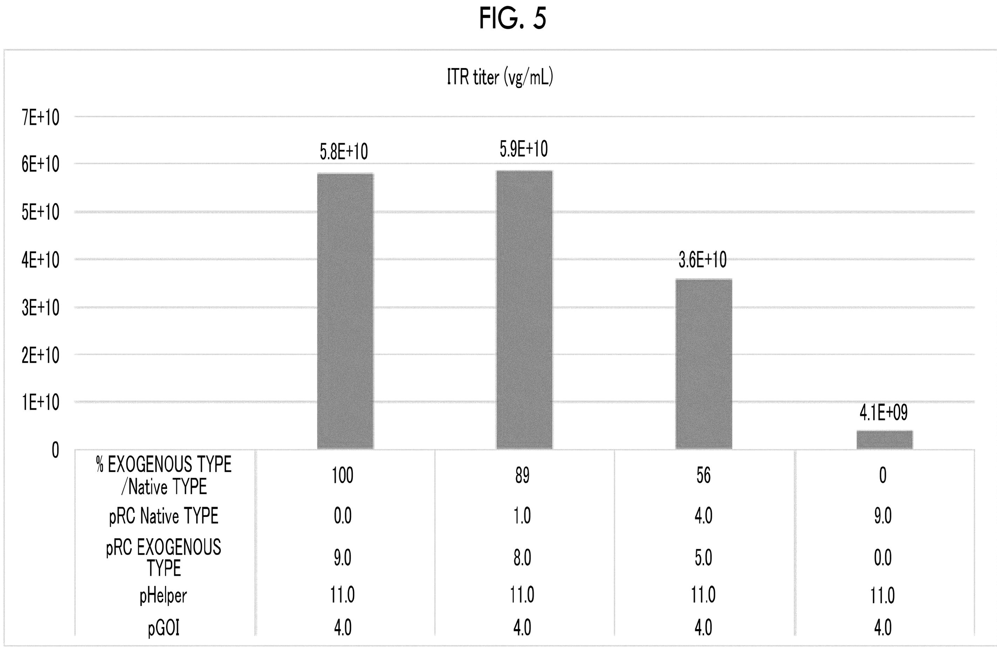

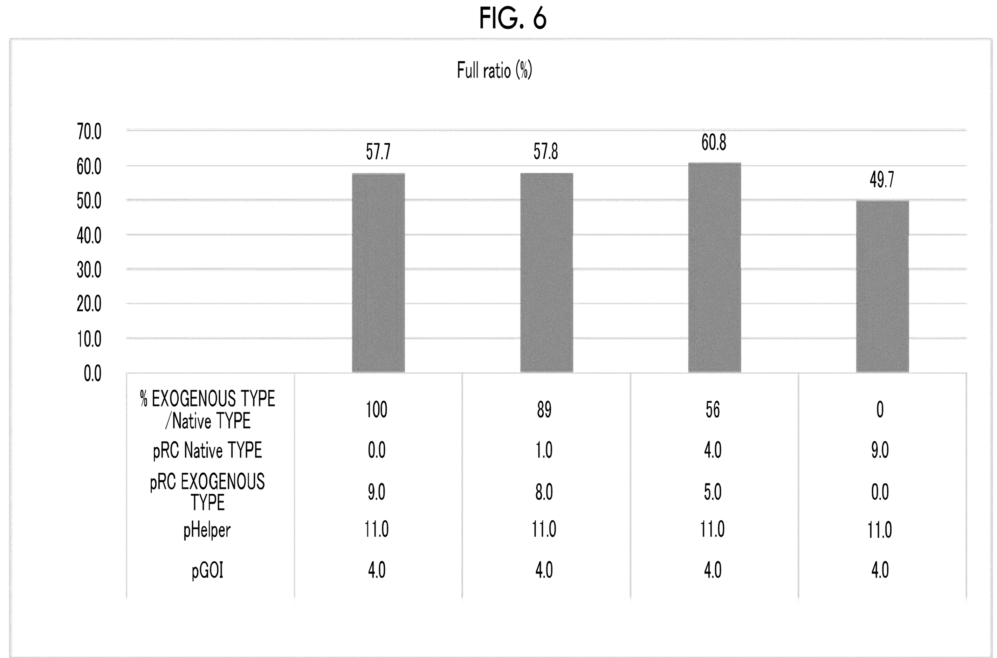

A transfection complex was prepared in the same manner as in Experimental Example 1, except that AAV6 serum type was used as the pRC Exogenous type and the plasmid was added in the following amounts described in Figs. 5 and 6.
pGOI (GOI plasmid): 4 µg
pRC Exogenous type (RepCap plasmid): 0 µg, 5 µg, 7 µg, 8 µg, or 9 µg
pRC Native type (Takara RepCap plasmid): 0 µg, 1 µg, 2 µg, 4 µg, or 9 µg
pHelper (Helper plasmid): 11 µg

Transfection and culture, AAV recovery for measuring genome titer, measurement of genome titer, AAV recovery for HPLC measurement, and HPLC measurement were performed in the same manner as in Experimental Example 1.

For Experimental Example 3, the measurement results of the genome titer are shown in Fig. 5, and the measurement results of the Full ratio are shown in Fig. 6.

### Experimental Example 4: HEK293 cells: AAV8

The cells were prepared in the same manner as in Experimental Example 1.

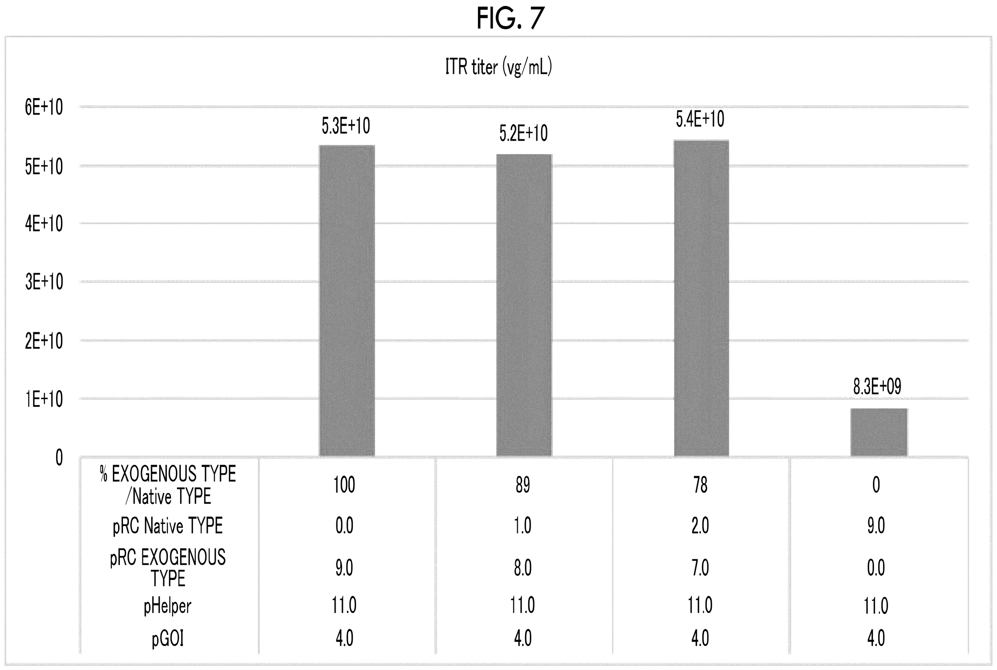

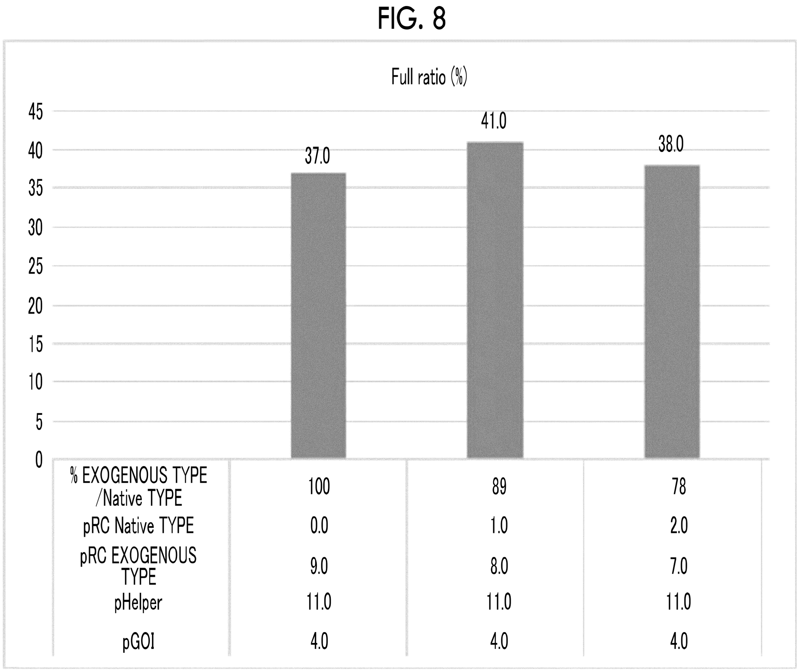

A transfection complex was prepared in the same manner as in Experimental Example 1, except that AAV8 serum type was used as the pRC Exogenous type and the plasmid was added in the following amounts described in Figs. 7 and 8.
pGOI (GOI plasmid): 4 µg
pRC Exogenous type (RepCap plasmid): 0 µg, 5 µg, 7 µg, 8 µg, or 9 µg
pRC Native type (Takara RepCap plasmid): 0 µg, 1 µg, 2 µg, 4 µg, or 9 µg
pHelper (Helper plasmid): 11 µg

Transfection and culture, AAV recovery for measuring genome titer, measurement of genome titer, AAV recovery for HPLC measurement, and HPLC measurement were performed in the same manner as in Experimental Example 1.

For Experimental Example 4, the measurement results of the genome titer are shown in Fig. 7, and the measurement results of the Full ratio are shown in Fig. 8.

### <Summary>

As shown in Figs. 1 to 8, a high Full ratio could be achieved by transferring a mixture containing at least a first vector containing a Rep gene and a Cap gene and a second vector containing a Rep gene and a Cap gene into cells.

### Experimental Example 5: Confirmation of REP expression by Western blotting

The cells were prepared in the same manner as in Experimental Example 1. Transfection was performed in the same manner as in Experimental Example 1, by adding, as a plasmid, pRC (RepCap plasmid) of AAV5 corresponding to the samples in Fig. 9 at the following amount ratio, in addition to 4 µg of pGOI (GOI plasmid) and 16 µg of pHelper (Helper plasmid). Cells were recovered after 72 hours from the transfection, and the REP protein was detected by Western blotting.
(Exogenous type plasmid sample) pRC Native type:pRC Exogenous type = 0 gg:16 µg
(1:1 mixed sample) pRC Native type:pRC Exogenous type = 8 µg:8 µg
(3:1 mixed sample) pRC Native type:pRC Exogenous type = 12 µg:4 µg
(7:1 mixed sample) pRC Native type:pRC Exogenous type = 14 µg:2 µg
(Native type plasmid sample) pRC Native type:pRC Exogenous type = 16 µg:0 µg [Sequence list] International application 22F01507W1JP23046847_1.xml based on International Patent Cooperation Treaty

## Claims

1. A method for manufacturing an adeno-associated virus, the method comprising:
a gene transfer step of transferring a first vector containing a Rep gene and a Cap gene and a second vector containing a Rep gene and a Cap gene into a cell; and
a cell culture step of culturing the cell into which gene transfer has been performed,
wherein the first vector is different from the second vector,
the Rep genes contained in the first vector and the second vector are full-length and of the same serum type, and
the Cap genes contained in the first vector and the second vector are full-length and of the same serum type.

2. The method according to claim 1,
wherein a promoter contained in the first vector is different from a promoter contained in the second vector.

3. The method according to claim 1 or 2,
wherein a promoter of the first vector is a wild-type promoter of an adeno-associated virus.

4. The method according to claim 3,
wherein the promoter of the first vector is a P5 promoter, a P19 promoter, or a P40 promoter.

5. The method according to claim 1 or 2,
wherein a promoter of the second vector is a non-adeno-associated virus-type promoter.

6. The method according to claim 1 or 2,
wherein a promoter of the second vector is a CMV promoter, a PGK promoter, or an EF1 promoter.

7. The method according to claim 1 or 2,
wherein the second vector further contains an IRES sequence and/or a 2A self-cleaving peptide sequence between the Cap gene and the Rep gene.

8. The method according to claim 1 or 2,
wherein the second vector is a vector containing a CMV promoter, a Cap gene, an EMCV sequence, and a Rep gene in this order.

9. The method according to claim 1 or 2,
wherein the first vector is a vector in which a production amount of a short form of a REP protein is larger than a production amount of a long form of the REP protein, and
the second vector is a vector in which a production amount of a long form of a REP protein is larger than a production amount of a short form of the REP protein.

10. The method according to claim 1 or 2,
wherein a use amount of the second vector is 5% or more with respect to a total use amount of the first vector and the second vector.

11. The method according to claim 1 or 2,
wherein the first vector and the second vector are plasmid vectors.

12. The method according to claim 1 or 2,
wherein in the gene transfer step, a third vector containing a virus helper gene and a fourth vector containing a gene for treatment or prevention are transferred into the cells.

13. The method according to claim 1 or 2,
wherein the cell is a HEK293 cell.

14. A set of vectors for manufacturing an adeno-associated virus, the set at least comprising:
a first vector containing a Rep gene and a Cap gene; and
a second vector containing a Rep gene and a Cap gene,
wherein the first vector is different from the second vector,
the Rep genes contained in the first vector and the second vector are full-length and of the same serum type, and
the Cap genes contained in the first vector and the second vector are full-length and of the same serum type.
